# EUROPEAN PATENT APPLICATION

(11) **EP 3 858 913 A1**
(43) Date of publication of application: **04.08.2021**
(21) Application number: 19865785.0
(22) Date of filing: 09.08.2019
(51) Int. Cl.: C08L 39/06, C08L 75/08, C12Q 1/00

(54) **POLYMER BLEND FOR CONTROLLING BLOOD GLUCOSE INFLUX, AND CONTINUOUS GLUCOSE MONITORING BIOSENSOR COMPRISING SAME**

(30) Priority: 27.09.2018 KR 20180115323
(71) Applicant: i-Sens, Inc., Seoul 06646 (KR)
(72) Inventor: JEONG, In Seok, Seoul 01060 (KR); LEE, Jinseon, Namyangju-si, Gyeonggi-do 12013 (KR); YANG, Hyunhee, Seoul 07220 (KR); KANG, Young Jea, Seoul 01213 (KR); SHIN, Jae Ho, Seoul 01405 (KR); HEO, Min, Seoul 04746 (KR); JEONG, Hee June, Seoul 02788 (KR)
(74) Representative: Arnold & Siedsma
(86) International application number: PCT/KR2019/010126
(87) International publication number: WO 2020/067641

(57) **Abstract**

The present disclosure relates to a continuous glucose monitoring biosensor, and more specifically, to: a biosensor which controls the flow of blood glucose flowing into a body during implantation, thereby enabling continuous detection and quantification of the blood glucose with high sensitivity; and a continuous glucose monitoring device and method using same. When a polymer blend composition including a hydrophilic polymer and a hydrophobic polymer according to the present disclosure, a diffusion control membrane formed therefrom and a continuous glucose monitoring biosensor including the same are used, they have the advantage capable of controlling an excessive influx of blood glucose due to an increase in the rate of diffusion in the sensor, thus enabling manufacture of a sensor having excellent accuracy and durability.

## Description

### [TECHNICAL FIELD]

### Cross-Reference to Related Application(s)

This application claims the benefit of Korean Patent Application No. 10-2018-0115323 filed on September 27, 2018 with the Korean Intellectual Property Office, the disclosure of which is incorporated herein by reference in its entirety.

The present disclosure relates to a continuous glucose monitoring biosensor, and more particularly to a polymer blend which controls the flow of blood glucose flowing in a body during implantation, and a biosensor which contains the polymer blend, thereby enabling continuous detection and quantification of the blood glucose with high sensitivity.

### [BACKGROUND ART]

Diabetes is a serious disease in which blood glucose increases abnormally in the process of insulin production or utilization, and accompanies various acute and chronic complications resulting therefrom. In the United States, 9.6% (more than 20 million people) of the population aged 20 and older have diabetes, and it is estimated that there are more than 50 million pre-diabetic patients at high risk of developing diabetes (The 2005 US National Diabetes Fact Sheet). Direct medical and indirect expenditures attributable to diabetes in 2002 was estimated at $132 billion US dollars.

In the case of South Korea, according to the Korea National Health and Nutrition Examination Survey conducted by the Korea Disease Control and Prevention Agency in 2005, it was found that 9.0% of men and 7.2% of women aged 30 years or older were diabetic. According to '2007 Korean Diabetes Research Report' published by the Korean Diabetes Association, it has been found that the prevalence of diabetes is about 8%, and 10% of new patients occurs every year, and diabetes-related medical expenses account for about 20% (about 3 trillion won) of health insurance costs. Considering the recent rapid increase in the number of diabetic patients, which is currently estimated at 4-5 million, it can reach 10 million 10-20 years later.

Diabetes, which is a global problem like this, is predicted to further increase in its prevalence due to the increase in the elderly population, living environmental factors and the like, and therefore, social and economic problems resulting therefrom are seriously rising. The diabetes is a disease that can be managed by controlling blood glucose levels, and therefore, due to the increase in the number of patients, the demand for self-monitoring blood glucose devices, which are mainly used for tracking and observing blood glucose levels, is increasing.

The disease management of such diabetes relies on closed-loop insulin delivery, and this is made possible by utilizing a continuous glucose monitoring (CGM) system that delivers signals directly to an insulin tank. However, since conventional enzyme sensors that collect blood from a fingertip have a restricted measurement frequency, they cannot be used for the CGM. In order to solve these problems, an improved version of an enzyme sensor that can be attached to the body and minimizes invasion has been recently developed. In the case of such a continuous blood glucose monitoring enzyme sensor, it has a diffusion control membrane used to regulate or limit the amount of glucose flowed in an in-vivo sample.

However, unless such a sensor is carefully designed from a physiological point of view, the glucose sensor may not respond sufficiently in an elevated glucose concentration range. If the inflow amount of glucose increases in the outermost layer of the biosensor system, a problem arises that such sensor measurement results may not be sufficient to reflect the glucose concentration in the tissue. Further, in the case of a continuous blood glucose monitoring sensor device mounted on a human body, the rate of supply (inflow) of glucose is further increased by convection caused by the stirring effect as a person continuously moves.

For example, when the concentration of glucose rises, it becomes too high in glucose permeability and can generate a current that is in the same plateau state as the glucose concentration rises above normal. Furthermore, when the diffusion rate of glucose in the sensor increases due to inflow, there may be a problem that not only the lifespan is shortened, but also the linearity is lowered.

However, in the case of a conventional diffusion control membrane, when the temperature rises by the use of polyvinylpyrrolidone (PVP), polyvinylimidazole (PVI), etc. there is a problem that the influx of blood glucose increases, making accurate measurement of blood glucose difficult, and thus, the temperature must be corrected.

In this regard, U.S. Patent No. 9,085,790 discloses an analyte sensor that is not affected by temperature, the analyte sensor including a working electrode, a counter electrode, a sensing layer disposed on the working electrode, and a stacked membrane structure which includes a first membrane having a negative temperature coefficient and a second membrane having a positive temperature coefficient, and has an analyte permeability that is substantially temperature independent, but does not disclose or imply any polymer blend technique that utilizes a water uptake rate of the polymer or has a specific water uptake rate.

On the other hand, U.S. Patent No. 7,613,491 relates to an implantable glucose sensor using a silicon-based membrane. In order to solve the measurement problem that the use period of the implantable glucose sensor is short and the accuracy of blood glucose is reduced, this patent discloses a device for an implantable analytical sensor further including an enzyme layer, in which a diffusion-resistant layer containing a silicone elastomer and a polyethylene oxide and polypropylene oxide copolymer is placed between the enzyme layer and the tissue, but it does not disclose or imply any polymer blend technique that utilizes a water uptake rate of the polymer or have a specific water uptake rate.

Therefore, the present inventors have made intensive research efforts to overcome the problems of the prior art, and as a result, found that a diffusion control membrane is produced using a polymer blend in which a hydrophilic polymer and a hydrophobic polymer are combined in a specific ratio, it is possible to develop an accurate and durable continuous blood glucose monitoring sensor that significantly regulates glucose influx in the sensor without being affected by various factors such as temperature, thereby completing the present disclosure.

### [DETAILED DESCRIPTION OF THE INVENTION]

### [Technical Problem]

The present invention has been designed to solve the above-mentioned problems, and therefore, it is an object of the present disclosure to provide a polymer blend composition for a diffusion control membrane of a continuous blood glucose monitoring sensor including a hydrophilic polymer and a hydrophobic polymer.

It is another object to provide a diffusion control membrane for a continuous blood glucose monitoring sensor composed of the polymer blend composition for the diffusion control membrane.

Another object is to provide a continuous blood glucose monitoring sensor including the diffusion control membrane.

### [Technical Solution]

In one embodiment, the present disclosure relates to a polymer blend composition for a diffusion control membrane of a continuous blood glucose monitoring sensor including a hydrophilic polymer and a hydrophobic polymer. Preferably, the hydrophilic polymer and the hydrophobic polymer may each contain one or more types, preferably one to three types. Specifically, the polymer blend composition for a diffusion control membrane of a continuous blood glucose monitoring sensor according to the present disclosure is characterized by particularly combining a hydrophilic polymer and a hydrophobic polymer having a specific range of water uptake rate. The term "water uptake rate" refers to a ratio at which the polymer allows external water to permeate its inside, and refers to the weight of water permeated with respect to the weight of the polymer.

Further, in a preferred embodiment, the polymer blend composition according to the present disclosure is characterized by having an amount of water uptake of 15 to 90% after 3 hours when immersed in 20 mL of PBS buffer.

The hydrophilic polymer contained in the blend composition of the present disclosure refers to a polymer capable of increasing the influx of water into the body and the influx of glucose contained in the water. Preferably, the hydrophilic polymer may be used without limitation as long as it has a water uptake rate of 10 to 200% based on the dry weight of the polymer.

For example, the hydrophilic polymer may be polyvinylpyrrolidone, an aliphatic polyether-based thermoplastic polyurethane, or a combination thereof, but can be used without limitation as long as it has the above water uptake rate. The aliphatic polyether-based thermoplastic polyurethane includes, for example, one or more selected from the group consisting of HP-60D-20 (Lubrizol), HP-60D-35 (Lubrizol), HP-60D-60 (Lubrizol), HP-93A-100 (Lubrizol), SP-80A-150 (Lubrizol), which are known under the trade name of Tecophilic™, and well known to have a water uptake rate in the above range; and polyvinylpyrrolidone, but is not limited thereto.

The hydrophilic polymer may be contained in an amount of 26 to 70% by weight based on the total weight of the blend composition of the present disclosure. When the hydrophilic polymer is contained in an amount of less than 26% by weight, the influx of glucose in the sensor is excessively limited, which is not preferable. When the hydrophilic polymer is contained in excess of 70% by weight, the influx of glucose is excessively increased, which is also not preferable.

On the other hand, the blend composition of the present disclosure contains a hydrophobic polymer, which refers to a polymer capable of reducing the influx of water into the body and the influx of glucose contained in the water. Preferably, the hydrophobic polymer may be used without limitation as long as it has a water uptake rate of 0 to 10% based on the dry weight of the polymer.

For example, the hydrophobic polymer is an aliphatic polyether-based thermoplastic polyurethane and may include SG80A (Lubrizol), SG93A (Lubrizol), SG60D (Lubrizol), EG72D (Lubrizol), and EG80A (Lubrizol), which are known under the trade name of Tecoflex™ and have a water uptake rate in the above range and thus are well known as a hydrophobic polymer, but is not limited thereto.

The hydrophobic polymer may be contained in an amount of 30 to 74% by weight based on the total weight of the blend composition of the present invention. When the hydrophobic polymer is contained in an amount of less than 30% by weight, the influx of glucose in the sensor is excessively increased, which is not preferable. When the hydrophobic polymer is contained in an amount of greater than 74% by weight, the influx of glucose is excessively reduced, which is also not preferable.

The polymer blend composition for the diffusion control membrane of the continuous blood glucose monitoring sensor according to the present disclosure has the feature that the hydrophilic polymer and the hydrophobic polymer are blended in a specific range, thereby allowing glucose to flow into the sensor at an appropriate diffusion rate while minimizing the influence of factors affecting the inflow and diffusion rate of glucose, such as temperature.

Further, unlike plural polymers with hydrophilic and hydrophobic segments, etc., the present polymer blend composition has the advantage capable of flexibly changing the content or type of the polymer as needed, and thus having high selectivity, and protecting an internal layer such as a sensing membrane from an external environment.

In another aspect, the present disclosure relates to a diffusion control membrane for a continuous blood glucose monitoring sensor composed of a polymer blend composition for a diffusion control membrane of the continuous blood glucose monitoring sensor, and a continuous blood glucose monitoring sensor including the same.

The continuous blood glucose monitoring sensor is preferably an electrochemical sensor for measuring blood glucose.

In the configuration of such a continuous blood glucose monitoring sensor, the present disclosure may include, for example, an electrode, an insulator, a substrate, a sensing layer including the electron transfer mediator and an oxidoreductase, a protection layer, and the like.

The sensing membrane may include an enzyme capable of oxidizing and reducing a liquid biological sample and the oxidation-reduction polymer. It relates to a sensing membrane for electrochemical biosensors including an electron transport medium. Oxidoreductase is a generic term for an enzyme that catalyzes the oxidation-reduction reaction in a living body, and in the case of a target material to be measured in the present disclosure, for example, a biosensor, it means an enzyme that is reduced by reacting with a target material to be measured. The enzyme reduced in this way reacts with the electron transfer mediator, and signals such as current change generated at the time is measured to quantify the target material. The oxidoreductase usable in the present disclosure may be at least one selected from the group consisting of various dehydrogenases, oxidases, esterases, and the like. Depending on the oxidation-reduction or detection target material, an enzyme including the target material as a substrate may be selected and used among enzymes belonging to the enzyme group.

More specifically, the oxidoreductase may be one or more selected from the group consisting of glucose dehydrogenase, glutamate dehydrogenase, glucose oxidase, cholesterol oxidase, cholesterol esterase, lactate oxidase, ascorbic acid oxidase, alcohol oxidase, alcohol dehydrogenase, bilirubin oxidase, and the like.

Meanwhile, the oxidoreductase may also include a cofactor that plays a role of storing hydrogen deprived by the oxidoreductase from the target material to be measured (e.g., target material), and example thereof may be one or more selected from the group consisting of flavin adenine dinucleotide (FAD), nicotinamide adenine dinucleotide (NAD), pyrroloquinoline quinone (PQQ), and the like.

Further, the sensing membrane may further include at least one additive selected from the group consisting of a crosslinking agent, a surfactant, a water-soluble polymer, a quaternary ammonium salt, a fatty acid, a thickener and the like, in order to perform the role of a dispersant during dissolution of reagents, an adhesive during preparation of reagents, a stabilizer during long-term storage, and the like.

In the case of an electrode, it may include two types of electrodes such as a working electrode and a counter electrode, and may also include three types of electrodes such as a working electrode, a counter electrode, and a reference electrode. In one embodiment, the biosensor according to the present disclosure may be an electrochemical biosensor manufactured by applying a reagent composition containing an electron transfer medium and an enzyme capable of oxidizing and reducing a liquid biological sample to a substrate having at least two, preferably two or three electrodes, followed by drying. For example, a planar electrochemical biosensor is provided in which in the electrochemical biosensor, the working electrode and the counter electrode are provided on opposite surfaces of the substrate, a sensing membrane containing an oxidation-reduction polymer according to the present disclosure is stacked on the working electrode, and insulators and diffusion membranes are sequentially stacked on both surfaces of the substrate provided with the working electrode and the counter electrode.

As a specific embodiment, the substrate may be composed of one or more materials selected from the group consisting of polyethylene terephthalate (PET), polycarbonate (PC), and polyimide (PI).

Further, carbon, gold, platinum, silver or silver/silver chloride electrodes may be used as the working electrode.

Further, in the case of an electrochemical biosensor having two electrodes, since the counter electrode performs up to the role of a reference electrode, gold, platinum, silver or silver/silver chloride electrodes can be used as the counter electrode, and in the case of a three-electrode electrochemical biosensor including up to a reference electrode, gold, platinum, silver, or silver/silver chloride electrodes may be used as a reference electrode, and a carbon electrode may be used as a counter electrode.

As a non-limiting example, in the case of a two-electrode, silver chloride or silver may be used because the counter electrode performs up to the role of the reference electrode. In the case of a three-electrode, silver chloride or silver may be used as the reference electrode, and a carbon electrode may be used as the counter electrode.

### [ADVANTAGEOUS EFFECTS]

When a polymer blend composition including a hydrophilic polymer and a hydrophobic polymer according to the present disclosure, a diffusion control membrane made of the same, and a biosensor for continuous blood glucose measurement including the same are used, it has the advantage of controlling the influx due to an increase in the diffusion rate of excessive blood glucose into the sensor, thereby enabling manufacture of a sensor having excellent accuracy and durability.

### [BRIEF DESCRIPTION OF THE DRAWINGS]

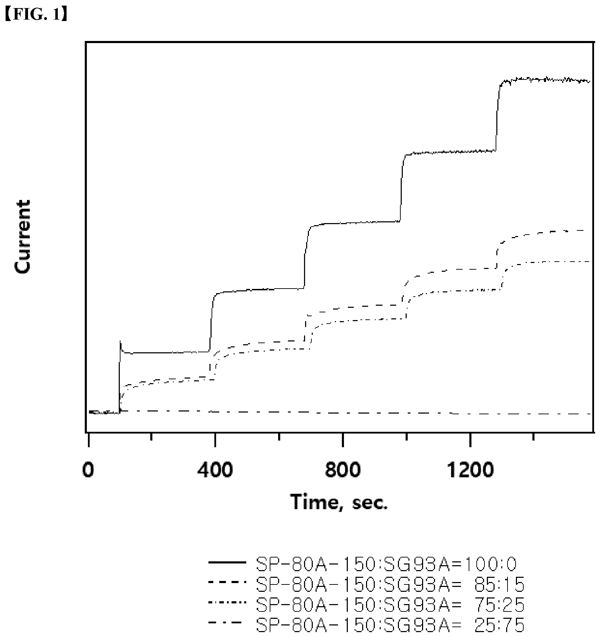

FIG. 1 is a result of confirming the intensity of current over time of a biosensor for continuous blood glucose measurement including a diffusion control membrane produced using the polymer blend composition (SP-80A-150+SG93A) according to the present disclosure, each prepared in various ratios of hydrophilic and hydrophobic polymers.

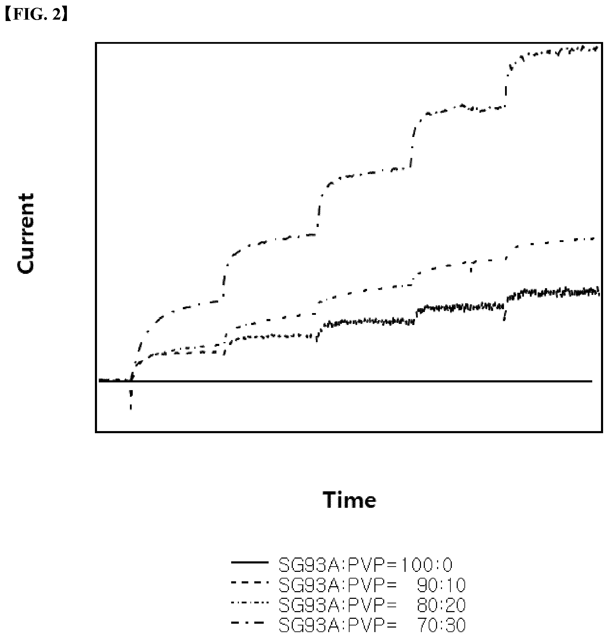

FIG. 2 is a result of confirming the intensity of current over time of a biosensor for continuous blood glucose measurement including a diffusion control membrane produced using the polymer blend composition (PVP+SG93A) according to the present disclosure, each prepared in various ratios of hydrophilic and hydrophobic polymers.

### [DETAILED DESCRIPTION OF THE EMBODIMENTS]

Hereinafter, the present disclosure will be described with reference to examples. However, these examples are for illustrative purposes only, and the scope of the present disclosure is not limited thereto.

### [EXAMPLE]

### Example 1: Production of a diffusion control membrane consisting of a polymer blend composition containing hydrophilic and hydrophobic polymers, and confirmation of electrochemical signal 1

A diffusion control membrane made of a polymer blend composition containing hydrophilic and hydrophobic polymers according to the present disclosure was produced as follows.

For the thermoplastic polyurethane, SP-80A-150 (Lubrizol), a solution grade, medical grade aliphatic, polyether-based polymer, which is also known under the trade name of Tecophilic™, and exhibits a water uptake rate of 150% of dry weight, was used as the hydrophilic polymer, and SG93A (Lubrizol), a solution grade, medical grade aliphatic, polyether-based hydrophobic polymer, which is also known under the trade name TecoflexTM, was used as the hydrophobic polymer. 50 mg of each of the hydrophilic and hydrophobic polymers was weighed and dissolved in 1 mL of tetrahydrofuran (THF). These were mixed in a volume ratio of SP-80A-150:SG93A of (i) 100:0, (ii) 85:15, (iii) 75:25, (iv) 25:75, respectively, to prepare a polymer blend.

5 uL of the polymer blend was placed on the electrode to which the sensing membrane was fixed, and then dried in an oven for 24 hours to produce a diffusion control membrane. Using electrochemical measuring equipment (CHI1030), the electrode thus produced was immersed in 10 mM PBS, pH 7.4 solution and stabilized, and then the change in the current value due to the change in glucose concentration was measured using i-t measurement method. As for glucose, a signal was measured until the glucose concentration reached 0 ∼ 5 mM by using a method of injecting a certain amount of a high-concentration glucose solution into a PBS solution containing an electrode. The results are shown in FIG. 1. As can be seen in FIG. 1, when a volume ratio of hydrophilic polymer: hydrophobic polymer was 100:0, the change in the current value due to the change in glucose concentration was measured to be the largest, and as the proportion of the hydrophilic polymer decreased, the change in the current value decreased. It was found that when the volume ratio of the hydrophilic polymer: hydrophobic polymer was 25:75, the change in the current value due to the change in the glucose concentration was hardly observed.

### Example 2: Production of a diffusion control membrane consisting of a polymer blend composition containing hydrophilic and hydrophobic polymers, and confirmation of electrochemical signal 2

A diffusion control membrane made of a polymer blend composition containing hydrophilic and hydrophobic polymers according to the present disclosure was produced as follows.

Polyvinylpyrrolidone (PVP, Sigma Aldrich) was used as the hydrophilic polymer, and SG93A (Lubrizol) was used as the hydrophobic polymer. 100 mL of the SG93A was dissolved in 1 mL of tetrahydrofuran (THF), and 200 mg of PVP was dissolved in 1 mL of ethanol to prepare each solution. These solutions were mixed in a volume ratio of SG93A:PVP of (i) 100:0, (ii) 90:10, (iii) 80:20, (iv) 70:30, respectively, to prepare a polymer blend.

5 uL of this polymer blend was placed on the electrode to which the sensing membrane was fixed, and then dried in an oven for 24 hours to produce a diffusion control membrane. Using electrochemical measuring equipment (CHI1030), the electrode thus produced was immersed in 10 mM PBS, pH 7.4 solution and stabilized, and then the change in the current value due to the change in glucose concentration was measured using i-t measurement method. As for glucose, a signal was measured until the glucose concentration reached 0 ∼ 5 mM by using a method of injecting a certain amount of a high-concentration glucose solution into a PBS solution containing an electrode. The results are shown in FIG. 2. As can be seen in FIG. 2, when a volume ratio of hydrophilic polymer: hydrophobic polymer was 30:70, the change in the current value due to the change in glucose concentration was measured to be the largest, and as the proportion of the hydrophilic polymer increased, the change in the current value decreased.

### Example 3: Analysis of water uptake capacity of a diffusion control membrane made of a polymer blend composition containing hydrophilic and hydrophobic polymers

A polymer membrane containing a polymer blend mixed according to the composition of Table 1 below was prepared by the following preparation method.

**[Table 1]**

| | Stock description | | Composition | |
|---|---|---|---|---|
| ratio (wt%) | SP-80A-150 | SG-93A | SP-80A-150 (mL) | SG-93A (mL) |
| SP-80A-150 : SG-93A | | | | |
| 100:0 | 10wt% in THF | 10wt% in THF | 4 | 0 |
| 75:25 | | | 3 | 1 |
| 50:50 | | | 2 | 2 |
| 25:75 | | | 1 | 3 |
| 0:100 | | | 0 | 4 |

First, 1.7 g of each of the hydrophilic polymer and the hydrophobic polymer was dissolved in 1.7 mL THF and stirred while heating the temperature to 40°C to prepare a stock solution. Respective stock solutions were mixed according to the ratio, and then mixed in a rotator for about half a day. Thereafter, 1.5 mL of the mixed solution was dispensed on a Teflon plate according to the ratio. After dispensing, it was left at room temperature for 30 minutes and dried in an oven at 25°C overnight when the outer surface dried slightly. In order to prevent the solution from flowing down, it was left at room temperature and then placed in an oven.

The polymer membrane prepared as described above was immersed in 20 mL of PBS buffer (10 mM PBS pH 7.4 (137 mM NaCl, 3 mM KCl)), and the amount of water uptake was calculated as 0% at 0 hours, and the amount of water uptake after 3 hours and 70 hours was confirmed and shown in Table 2 below.

**[Table 2]**

| Confirmation of the amount of water uptake of the polymer blend | | | | | |
|---|---|---|---|---|---|
| | SP-80A-1 50:SG-93A (wt.%) | | | | |
| | 100:0 | 75:25 | 50:50 | 25:75 | 0:100 |
| 3h | 112% | 83% | 50% | 16% | 0% |
| 70h | 125% | 87% | 47% | 21% | 1% |

As can be seen in Table 2, it was confirmed that the water uptake capacity of the hydrophilic polymer progressed in the direction of reaching the limit of the uptake capacity after absorbing a certain ratio. And, when the membrane was prepared by blending the hydrophilic polymer and the hydrophobic polymer, it can be confirmed that as the proportion of hydrophilic polymer decreases, the water uptake capacity decreases proportionally. Through these properties, it could be expected that the degree of diffusion of glucose could be adjusted by adjusting the blend ratio of the hydrophilic polymer and the hydrophobic polymer.

## Claims

1. A polymer blend composition for a diffusion control membrane of a continuous blood glucose monitoring sensor comprising a hydrophilic polymer having a water uptake rate of 10 to 200% and a hydrophobic polymer having a water uptake rate of 0 to 10%.

2. The polymer blend composition for a diffusion control membrane of a continuous blood glucose monitoring sensor according to claim 1, wherein the hydrophilic polymer contains 26 to 70% by weight based on the total weight of the polymer blend composition.

3. The polymer blend composition for a diffusion control membrane of a continuous blood glucose monitoring sensor according to claim 1, wherein the hydrophobic polymer contains 30 to 74% by weight based on the total weight of the polymer blend composition.

4. The polymer blend composition for a diffusion control membrane of a continuous blood glucose monitoring sensor according to claim 1, wherein the hydrophilic polymer is polyvinylpyrrolidone (PVP), an aliphatic polyether-based thermoplastic polyurethane, or a combination thereof.

5. The polymer blend composition for a diffusion control membrane of a continuous blood glucose monitoring sensor according to claim 1, wherein when immersed in 20 mL of PBS buffer, the amount of water uptake after 3 hours is 15 to 90%.

6. A diffusion control membrane of a continuous blood glucose monitoring sensor comprising the polymer blend composition for a diffusion control film of the continuous blood glucose monitoring sensor of any one of claims 1 to 5.

7. A continuous blood glucose monitoring sensor comprising the diffusion control membrane of the continuous blood glucose monitoring sensor according to claim 6.
